(19) Europäisches Patentamt
European Patent Office
Office européen des brevets

(11) Publication number: 0 155 710 B1

# (12) EUROPEAN PATENT SPECIFICATION

(45) Date of publication of patent specification: 28.09.88

(21) Application number: 85200114.8

(22) Date of filing: 28.04.82

(60) Publication number of the earlier application in accordance with Art. 76 EPC: 0 064 384

(51) Int. Cl.[4]: C 07 C 143/11, C 07 C 149/18, C 07 C 149/267

## (54) Process for the preparation of derivatives of polyethoxypropanesulfonates.

(30) Priority: 30.04.81 US 259218

(43) Date of publication of application: 25.09.85 Bulletin 85/39

(45) Publication of the grant of the patent: 28.09.88 Bulletin 88/39

(84) Designated Contracting States: BE DE FR GB IT NL

(58) References cited:
GB-A-1 566 655
US-A-4 267 123

(73) Proprietor: MOBIL OIL CORPORATION
150 East 42nd Street
New York New York 10017 (US)

(72) Inventor: Schmitt, Kirk Douglas
217 N. Main Street
Pennington, NJ (US)

(74) Representative: Cooper, John Anthony et al
Mobil Court 3 Clements Inn
London WC2A 2EB (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 155 710 B1

Courier Press, Leamington Spa, England.

**Description**

This invention relates to the preparation of alkoxypropane sulfonates; more particularly, this invention relates to the preparation of alkoxypolyethoxypropane sulfonates.

GB—A—1566655 discloses a method of making ether sulfonates which method comprises reacting an alkoxylated alcohol with an alkyl halide in the presence of a strong base to form an allyl ether; separating the allyl ether; and reacting the allyl ether with sodium sulfite to form the ether sulfonate. An overall yield of about 70% is exhibited.

This invention seeks to provide a process for the production of ether sulfonates, particularly alkoxypolyethoxypropane sulfonates, in enhanced yield.

According, therefore, to the present invention there is provided a process for preparing a compound of the formula:

$$RY(C_2H_4O)_xCH_2CH_2CH_2SO_3Na$$

in which:

R represents an alkyl or alkaryl group;
Y represents an oxygen or sulfur atom; and
x has a value from 0 to 20,

which process comprises (i) reacting together an alcohol of the formula $RY(C_2H_4O)_xH$ (wherein R, Y and x are as herein defined), sodium hydroxide and an allyl halide or tosylate to form the corresponding allyl ether of the formula $RY(C_2H_4O)_xCH_2CH{=}CH_2$ (wherein R, Y and x are as herein defined) and (ii) reacting the allyl ether with sodium bisulfite characterised in that the first stage of the reaction is effected in from 50% to 70%, preferably from 40% to 60% such as about 50%, by weight aqueous sodium hydroxide in the presence of sufficient final product to function as a phase transfer agent: and in that the second stage is effected without isolation of the intermediate allyl ether.

This process has several inherent advantages over known processes for the production of propane sulfonates. First, it eliminates the use, and hence the cost of separation, recovery and recycle of organic solvent; secondly, since intermediate product isolation is unnecessary, the process can be carried out in a single reaction vessel; and thirdly, the product is obtained in a high yield (rarely below 90%).

The two-stage process of the present invention can be represented schematically as follows:

$$RY(C_2H_4O)_xH + NaOH + XCH_2CH = CH_2 \rightarrow RY(C_2H_4O)_xCH_2CH = CH_2 + NaX$$

$$RY(C_2H_4O)CH_2CH = CH_2 + NaHSO_3 \rightarrow RY(C_2H_4O)_xCH_2CH_2CH_2SO_3Na$$

R, Y and *x* having the meanings given above and X being halide or tosylate. R is preferably a $C_1$—$C_{16}$ alkyl or $C_7$—$C_{16}$ alkaryl group, especially a $C_{10}$—$C_{16}$ alkyl group, and *x* is preferably 2 to 6, especially 3. The reaction is suitably carried out at 0 to 55°C at ambient pressure in the presence of from 0.1 to 1 mole of final product per mole of alcohol $RY(C_2H_4O)_xH$. Any excess, unreacted allyl compound $XCH_2CH = CH_2$ may be distilled off at the end of the reaction.

The following Examples illustrate the invention.

The compounds identified as I, II and III in these Examples are identified as follows (n = 4.2):

I 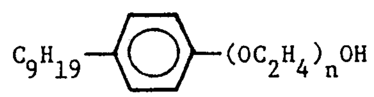

II 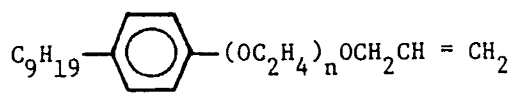

III 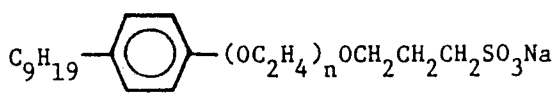

In these Examples HPLC was carried out on the reaction product by injecting samples into a Whatman ODS C—18 column and eluting at 2 ml/min with a solvent whose composition changed linearly from 70% methanol (30% $H_2O$) to 100% methanol over 15 minutes. Under these conditions, compound III had a retention volume from 3.6 to 6.1 ml, compound I has a retention volume of 14.4 ml and compound II had a retention volume of 18.8 ml.

Example

To a four-neck 250 ml flask were added 19.3 g allyl chloride (.252 mole), 10 g 51% NaOH (.128 mole), 20.3 g I (.050 mole) and 5.05 g III (.0092 mole). The mixture was heated to reflux and stirred mechanically under argon. Aliquots were removed periodically and analyzed by HPLC. The results were as follows:

| Reflux Time (hours) | % Reaction |
|---|---|
| 1 | 84 |
| 3 | 86 |
| 6 | 91 |
| 22 | 97 |

At the end of the reflux period, the excess allyl chloride was distilled off, the reaction mixture was cooled, air flowed through the flask at about 15 ml/min and 40 ml 95% ethanol were added. After stirring briefly to assure air saturation, a solution of 15.7 g $NaHSO_3$ and 3.6 g $Na_2SO_3$ in 42 ml $H_2O$ was added dropwise over 15 min. The temperature rose to 34°C and HPLC analysis after 3 hours indicated that less than 1% allyl ether II remained. An aliquot was stripped and analyzed by C—13 NMR; it showed 4% sulfite to be present so the overall conversion of I was 96% and the overall yield of propane sulfonate III was 92%.

The flask was cooled to 15°C, inorganics filtered off and the ethanol and water removed to give 33.5 g of pale yellow wax.

Comparative Example

Polyethyleneoxy alcohols are known to act themselves as phase transfer agents (see for example, *Journal of Organic Chemistry, 45,* 1095 (1980): thus, compound I catalyzes its own reaction at the beginning of the process. However, as it is converted to allyl ether II the reaction slows and stops.

19.3 g allyl chloride, 30.3 g alcohol I, and 10 g 51% NaOH were stirred and refluxed under argon and the reaction analyzed by HPLC as described in Example. The results were as follows:

| Reflux Time (hours) | % Reaction |
|---|---|
| 1 | 70 |
| 3 | 77 |
| 6 | 79 |
| 22 | 83 |

It will thus be apparent that where III is absent from the reaction medium lower conversions, and thus lower yields of desired product, are obtained.

**Claims**

1. A process for preparing a compound of the formula:

$$RY(C_2H_4O)_xCH_2CH_2CH_2SO_3Na$$

in which:

R represents an alkyl or alkaryl group;

Y represents an oxygen or sulfur atom; and

x has a value from 0 to 20,

which process comprises (i) reacting together an alcohol of the formula $RY(C_2H_4O)_xH$ (wherein R, Y and x are as herein defined), sodium hydroxide and an allyl halide or tosylate to form the corresponding allyl ether of the formula $RY(C_2H_4O)_xCH_2CH{=}CH_2$ (wherein R, Y and x are as herein defined) and (ii) reacting the allyl ether with sodium bisulfite characterised in that the first stage of the reaction is effected in from 50% to 70% by weight aqueous sodium hydroxide in the presence of sufficient final product to function as a phase transfer agent: and in that the second stage is effected without isolation of the intermediate allyl ether.

2. A process according to claim 1, in which R is an alkyl group having from 1 to 16 carbon atoms or an alkaryl group having from 7 to 16 carbon atoms and *x* is zero or has a value from 2 to 6.

3. A process according to claim 1 or claim 2, in which the first stage is carried out in the presence of from 0.1 to 1 mole of the final product per mole of alcohol $RY(C_2H_4O)_xH$.

**Patentansprüche**

1. Verfahren zur Herstellung einer Verbindung der Formel

$$RY(C_2H_4O)_xCH_2CH_2CH_2SO_3Na$$

worin:
R eine Alkyl- oder Alkarylgruppe darstellt,
Y ein Sauerstoff- oder Schwefelatom darstellt und
x einen Wert von 0 bis 20 hat,
wobei dieses Verfahren umfaßt: (i) die Reaktion eines Alkohols der Formel $RY(C_2H_4O)_xH$ (worin R, Y und x wie oben definiert sind), von Natriumhydroxid und einem Allylhalogenid oder -tosylat, um den korrespondierenden Allylether der Formel $RY(C_2H_4O)_xCH_2CH{=}CH_2$ (worin R, Y und x wie oben definiert sind) zu bilden, und (ii) Reaktion dieses Allylethers mit Natriumbisulfit, dadurch gekennzeichnet, daß die erste Reaktionsstufe in wässrigem, von 50 bis 70 Gew-%igem Natriumhydroxid in Gegenwart von ausreichend Endprodukt durchgeführt wird, um als Phasenübertragungsmittel zu wirken, und daß die zweite Stufe ohne Isolierung des Allylether-Zwischenproduktes durchgeführt wird.

2. Verfahren nach Anspruch 1, worin R eine Alkylgruppe mit von 1 bis 16 Kohlenstoffatomen oder eine Alkarylgruppe mit von 7 bis 16 Kohlenstoffatomen ist und x Null ist oder einen Wert von 2 bis 6 hat.

3. Verfahren nach Anspruch 1 oder 2, worin die erste Stufe in Gegenwart von 0,1 bis 1 Mol des Endproduktes pro Mol des Alkohols $RY(C_2H_4O)_xH$ durchgeführt wird.

**Revendications**

1. Un procédé de préparation d'un composé de formule:

$$RY(C_2H_4O)_xCH_2CH_2CH_2SO_3Na$$

dans laquelle:
R représente un groupe alkyle ou alcaryle;
Y représente un atome d'oxygène ou de soufre; et
x a une valeur de 0 à 20,
ledit procédé comprenant (i) la réaction ensemble d'un alcool de formule $RY(C_2H_4O)_xH$ (dans laquelle R, Y et x sont définis comme ci-dessus), avec l'hydroxyde de sodium et un halogénure ou tosylate d'allyle pour former l'éther allylique correspondant de formule $RY(C_2H_4O)_xCH_2CH{=}CH_2$ (dans laquelle R, Y et x sont définis comme ci-dessus) et (ii) la réaction de l'éther allylique avec le bisulfite de sodium, caractérisé en ce que la première étape de la réaction est effectuée avec 50% à 70% en poids d'hydroxyde de sodium aqueux en présence d'une quantité suffisante de produit final agissant comme agent de transfert de phase, et en ce que la seconde étape est effectuée sans isolement de l'éther allylique intermédiaire.

2. Un procédé selon la revendication 1, selon laquelle R est un groupe alkyle ayant de 1 à 16 atomes de carbone ou un groupe alcaryle ayant de 7 à 16 atomes de carbone et *x* est égal à 0 ou a une valeur de 2 à 6.

3. Un procédé selon la revendication 1 ou 2, selon laquellela première étape est conduite en présence de 0,1 à 1 mole de produit final par mole d'alcool $RY(C_2H_4O)_xH$.